# EUROPÄISCHE PATENTANMELDUNG

(11) **EP 0 742 271 A2**
(43) Veröffentlichungstag der Anmeldung: **13.11.1996**
(21) Anmeldenummer: 96107162.8
(22) Anmeldetag: 07.05.1996
(51) Int. Cl.: C09C 1/00, C09D 7/12, C08K 9/02, A61K 7/00

(54) **Farbpigmente**

(30) Priorität: 12.05.1995 DE 19516960
(71) Anmelder: MERCK PATENT GmbH, 64293 Darmstadt (DE)
(72) Erfinder: Ambrosius, Klaus, Dr., 64807 Dieburg (DE); Schraml-Marth, Matthias, Dr., 64673 Zwingenberg (DE)

(57) **Zusammenfassung**

Die vorliegende Erfindung betrifft Farbpigmente basierend auf Substraten, die mit einer dotierten Schicht aus Eisen(III)oxid beschichtet sind, sowie deren Herstellungsverfahren und Verwendung, insbesondere in Wasserlacksystemen.

## Beschreibung

Die vorliegende Erfindung betrifft Farbpigmente basierend auf Substraten, die mit einer dotierten Schicht aus Eisen(III)oxid beschichtet sind, sowie deren Herstellungsverfahren und Verwendung, insbesondere in Wasserlacksystemen.

Eisenoxidhaltige Pigmente sind vielfach in der Literatur beschrieben. Perlglanzpigmente, deren Goldton durch Zusatz von Eisenoxiden verstärkt wird, sind aus der DE 14 67 468 bekannt. Ein spezielles Verfahren zur Herstellung eines Glanzpigmentes mit Eisenoxidzusatz wird in der P 19 59 998 beschrieben. Zusätzlich ist hier die eisenoxidhaltige Schicht noch mit einer Titan- und/oder Zirkondioxidschicht überzogen. Das in diesen Glanzpigmenten enthaltene Eisen(III)oxid wird im sauren Bereich aus Eisen(III)salzlösungen ausgefällt. Aus der DE 22 44 298 sind goldfarbene Pigmente auf Basis von Titandioxid beschichteten Glimmerschuppen bekannt, die mit einer Fe₂O₃-Schicht nachbeschichtet sind, hergestellt durch Oxidation von Eisen(II)hydroxid. Aus der SU 16 999 930 A1 sind Rotpigmente mit einem erhöhtem Perlmuttglanz auf Basis von Glimmerplättchen bekannt, die mit Fe₂O₃ und B₂O₃ beschichtet sind.

In der DE 41 35 742 A1 werden mit B₂O₃ dotierte Eisenoxidpigmente beschrieben, die sich durch ihre besonders glatte Pigmentoberfläche auszeichnen. Die nach dem Nitrobenzolverfahren hergestellten Magnetitpigmente können durch nachträgliches Glühen an der Luft in Rotpigmente verschiedener Farbnuancen überführt werden. Aus der US 5,273,576 sind Perlglanzpigmente bekannt, wobei Glimmer mit einer mit SnO₂, ZnO oder ZrO₂ dotierten Eisen(III)oxidschicht belegt ist, die sich durch ihr erhöhtes Chroma auszeichnen.

Die aus dem Stand der Technik bekannten Eisenoxidpigmente zeichnen sich durch ihren hohen Farbwert und ihre Wetterbeständigkeit bei der Verwendung in nicht wäßrigen Lacksystemen aus. In wäßrigen Lacksystemen, die immer mehr an Bedeutung gewinnen, insbesondere in der Automobilindustrie, zeigen die bekannten Eisenoxidpigmente aber alle den Nachteil, daß sie zur Bildung von mikrofeinen Bläschen im Lackfilm führen. Diese Bläschen führen zu einer erhöhten Lichtstreuung und beeinflussen damit nachteilig Farbe und Glanz des pigmentierten Wasserlacksystems.

Ein weiterer Nachteil ist die Verminderung der Abbildungsklarheit (DOI) sowie die Verschlechterung des Regenerationsvermögens der pigmentierten Lackschicht.

Aufgabe der vorliegenden Erfindung war es daher modifizierte eisenoxidhaltige Pigmente zu finden, die ohne Verlust ihrer Eigenschaften in Wasserlacksysteme gut dispergierbar sind und keine der oben genannten Nachteile aufweisen.

Überraschenderweise wurde nun gefunden, daß Substrate, die mit einer SiO₂ und ZrO₂ dotierten Eisen(III)oxidschicht überzogen sind, sich durch ihren hohen Farbwert und ihren Glanz auszeichnen und sich ohne Probleme in Wasserlacksysteme einarbeiten lassen.

Gegenstand der Erfindung sind daher Farbpigmente auf Basis von mit Eisen(III)oxiden beschichteten Substraten, dadurch gekennzeichnet, daß die Eisenoxidschicht mit ZrO₂ und SiO₂ dotiert ist.

Gegenstand der Erfindung ist weiterhin ein Verfahren zur Herstellung der erfindungsgemäßen Pigmente, das sich dadurch auszeichnet, daß man zu der wäßrigen Substratsuspension nacheinander oder gleichzeitig eine wäßrige Eisen(III)salzlösung, Zirkoniumsalzlösung und eine wasserlösliche Siliziumverbindung unter Bedingungen zugibt, die zur Abscheidung der entsprechenden Hydroxide bzw. Oxide führen, und anschließend das Pigment abtrennt, wäscht, trocknet und bei Temperaturen > 500 °C glüht.

Geeignete Basissubstrate für die Beschichtung sind einerseits opake und andererseits transparente plättchenförmige oder nicht-plättchenförmige Substrate. Bevorzugte Substrate sind Schichtsilikate sowie mit Metalloxiden beschichtete plättchenförmige Materialien. Insbesondere geeignet sind Glimmer, Talkum, Kaolin, Wismutoxichlorid, Glas-, SiO₂- oder Keramikflakes, synthetische trägerfreie Plättchen oder andere vergleichbare Materialien. Daneben kommen auch Metallplättchen, wie z.B. Aluminiumplättchen oder plättchenförmige Metalloxide, wie z.B. plättchenförmiges Eisenoxid und Glimmerbeschichtungen mit farbigen oder farblosen Metalloxiden wie TiO₂, Fe₂O₃, SnO₂, Cr₂O₃, ZnO und anderen Metalloxiden, allein oder in Mischung in einer einheitlichen Schicht oder in aufeinanderfolgenden Schichten zur Anwendung. Diese als Perlglanzpigmente bekannten Pigmente sind z.B. aus den deutschen Patenten und Patentanmeldungen 14 67 468, 19 59 998, 20 09 566, 22 14 545, 22 15 191, 22 44 298, 23 13 331, 25 22 572, 31 37 808, 31 37 809, 31 51 343, 31 51 354, 31 51 355, 32 11 602 und 32 35 017 bekannt.

Die plättchenförmigen Substrate haben in der Regel eine Dicke zwischen 0,1 und 5 µm und insbesondere zwischen 0,2 und 4,5 µm. Die Ausdehnung in den beiden anderen Bereichen beträgt üblicherweise zwischen 1 und 250 µm, insbesondere zwischen 2 und 200 µm.

Zur Herstellung der erfindungsgemäßen Pigmente wird zunächst eine wäßrige Suspension des Substrats hergestellt. Zu der Suspension werden gleichzeitig oder vorzugsweise nacheinander eine wäßrige Eisen(III)salzlösung, eine wäßrige Zirkoniumsalzlösung und eine wäßrige Silikatlösung zudosiert, wobei der pH-Wert des Reaktionsgemisches durch gleichzeitige Zugabe einer Säure oder Base in einem Bereich gehalten wird, der die Metallsalzhydrolyse bewirkt. Dabei wird das Fe₂O₃ und ZrO₂ und SiO₂ bzw. die entsprechenden Hydroxide entweder nacheinander oder gleichzeitig auf die Substratoberfläche aufgefällt. Die Temperatur der Reaktionslösung ist nicht sehr kritisch und beträgt üblicherweise 20-80 °C. Die Fällung der Oxide bzw. Hydroxide wird in der Regel bei einem pH-Wert zwischen 3 und 12 vorgenommen. Durch die Zugabe von Säuren, vorzugsweise Mineralsäuren wie z.B. HCl, HNO₃, H₂SO₄, und Basen wie z.B. NaOH, KOH, Na₂CO₃, K₂CO₃, NH₃ wird der pH-Wert leicht auf konstantem Niveau gehalten.

Nach dem Abtrennen, Waschen und Trocknen der beschichteten Substrate werden die Pigmente bei Temperaturen > 500 °C, vorzugsweise bei 800-850 °C, geglüht. Die Glühtemperatur hängt in der Regel von der aufgefällten Schichtdicke ab; die Glühtemperatur kann von einigen Minuten bis zu mehreren Stunden, vorzugsweise jedoch zwischen 20 und 120 Minuten, betragen.

Als Metallsalze aus denen die Hydroxide bzw. Oxide gefällt werden können, sind alle wasserlöslichen Salze verwendbar, die durch Basen oder Säuren hydrolysierbar sind. Geeignete Eisen(III)salze sind insbesondere die Eisen(III)halogenide, -nitrate, -sulfate, vorzugsweise Eisen(III)chlorid.

Die Zirkoniumverbindungen werden vorzugsweise in Form der Chloride ZrCl₄ oder als basisches Chlorid ZrOCl₂ eingesetzt.

Das Siliziumdioxid wird in Form einer wasserlöslichen anorganischen Siliziumverbindung der Suspension zugesetzt. Geeignet sind die unter dem Namen ''Wasserglas'' im Handel erhältlichen wäßrigen Lösungen von Alkalisilikaten, wie z.B. Kaliwasserglas und Natronwasserglas.

Alle denkbaren ZrO₂:SiO₂-Verhältnisse können bei der Dotierung der Eisen(III)oxidschicht verwendet werden. Ein positiver Einfluß auf Farbstärke und Glanz ist besonders dann zu beobachten, wenn das Verhältnis ZrO₂ zu SiO₂ 10:1 bis 0,1:1, vorzugsweise 5:1 bis 0,5:1 beträgt. Der Anteil der Dotierung in der Eisen(III)oxidschicht beträgt 0,1 bis 10 Gew.%, vorzugsweise 0,1 bis 5 Gew.%, insbesondere 0,1 bis 3 Gew.%.

Die nach dem erfindungsgemäßen Verfahren hergestellten Farbpigmente zeichnen sich durch ihre hohe Farbstärke, ihren Glanz und ihr hohes Deckvermögen aus. Sie sind mit einer Vielzahl von Farbsystemen kompatibel, vorzugsweise aus dem Bereich der Lacke, Farben und Druckfarben.

Aufgrund ihrer hohen Licht- und Witterungsbeständigkeit eignen sie sich hervorragend für Autolacke, insbesondere für Lacke auf Basis wäßriger Systeme.

Gegenstand der Erfindung ist somit auch die Verwendung der erfindungsgemäßen Eisenoxidpigmente in Formulierungen wie Farben, Lacken, Druckfarben, Kunststoffen und Kosmetikpräparaten.

In der Regel werden die Pigmente in die Formulierung in Mengen bis zu höchstens 30 %, vorzugsweise 0,5 bis 10 %, eingesetzt.

Gegenstand der Erfindung sind weiterhin Formulierungen, die die erfindungsgemäßen Pigmente enthalten.

Die im folgenden angegebenen Beispiele sollen die Erfindung erläutern, ohne sie zu begrenzen.

### Beispiel 1

Eine Suspension von 10 kg Glimmer der Teilchengröße 10-50 µm in 150 l Wasser wird unter Rühren auf 75 °C erwärmt. Der pH-Wert wird mit 5%iger HCl auf 3 eingestellt. Anschließend wird eine wäßrige FeCl₃-Lösung (Fe-Gehalt 3 Gew.%) zugegeben, wobei der pH-Wert mit ca. 30%iger NaOH auf 3,0 gehalten wird. Nach Erreichen der roten Interferenzfarbe wird die Zugabe der FeCl₃-Lösung abgebrochen und man rührt 15 Min. nach.

Nach dem Einstellen des pH-Wertes auf 3,2 mit 5%iger NaOH wird eine Lösung bestehend aus 175 g ZrOCl₂ x 8 H₂O in 15 l Wasser innerhalb einer Stunde zugegeben. Man rührt weitere 15 Min. nach und stellt den pH-Wert mit 5%iger NaOH auf 9 ein. Innerhalb einer Stunde wird eine wäßrige Natronwasserglaslösung [180 ml Natronwasserglas (27 Gew.%) in 15 l Wasser] zudosiert, wobei der pH-Wert mit 5%iger HCl konstant gehalten wird. Nach beendeter Zugabe wird der pH-Wert mit 5%iger HCl auf 6,5 eingestellt, und die Suspension wird weitere 15 Min. gerührt. Das Produkt wird abfiltriert, mit Wasser gewaschen, getrocknet und bei 850 °C geglüht.

### Beispiel 2 - Vergleichsbeispiel

Eine Suspension von 10 kg Glimmer der Teilchengröße 10-50 µm in 150 l Wasser wird auf 75 °C erhitzt. Der pH-Wert wird mit 5%iger HCl auf 3,0 eingestellt. Anschließend wird eine wäßrige FeCl₃-Lösung (Fe-Gehalt 3 Gew.%) zudosiert, wobei der pH-Wert durch gleichzeitige Zugabe von 30%iger NaOH konstant gehalten wird. Nach Erreichen der roten Interferenzfarbe wird die Zugabe der FeCl₃-Lösung abgebrochen, und man rührt weitere 15 Min. nach.

Der pH-Wert wird mit 5%iger NaOH auf 5,0 eingestellt, und die Suspension wird weitere 15 Min. gerührt. Das Produkt wird abfiltriert, mit Wasser gewaschen, getrocknet und bei 850 °C geglüht.

Die Beurteilung der Pigmente erfolgt nach dem Kondenswassertest.

Die Pigmente aus Beispiel 1 und dem Vergleichsbeispiel werden in einen handelsüblichen Wasserlack eingearbeitet und die Prüfproben per Spritzapplikation auf ein Metallblech hergestellt.

Die Prüfung erfolgt nach DIN 50017 (Kondenswasserprüfung-Konstant-Klima).

Testdauer: 240 h bei 40 °C

Die Beurteilung des Quellungsvorganges erfolgt visuell in Anlehnung an DIN 53230. In der Bewertungsskala bedeuten die Kennzahlen 0 "nicht verändert" und 5 "sehr stark verändert".

Die Beurteilung des Blasengrades erfolgt visuell nach DIN 53209. "m" bedeutet die Häufigkeit der Blasen je Flächeneinheit und "g" bedeutet Größe der Blasen. Die Bewertungsskala reicht von 0 (sehr gut) bis 5 (sehr schlecht).

Die Glanzbeurteilung erfolgt mit Hilfe eines Reflektometers nach DIN 67530.

Die Abbildungsklarheit (DOI) wird nach ASTM EU 30-91 bestimmt.

Die Testergebnisse sind Tabelle 1 zu entnehmen:

**Tabelle 1**

| Beispiel | Quellung | Blasengrad | Gitterschmitt-Test | Glanzbeurteilung bei 20° in % | Abbildungsklarheit in % |
|---|---|---|---|---|---|
| 1 | 1-2 | m 0/g 0 | Gt 0 | 94 | 97 |
| 2 | 4 | m 3-4/g 0-1 | Gt 0 | 90 | 59 |
| Nullprobe (ohne Pigment) | 1 | m 0/g 0 | Gt 1 | 97 | 94 |

Aus Tabelle 1 ist ersichtlich, daß das erfindungsgemäße Pigment im Wasserlack die Bildung feiner Mikrobläschen unterdrückt, die Abbildungsklarheit erhöht und im Vergleich zum unbehandelten Pigment den Glanz weit weniger beeinflußt.

## Patentansprüche

1. Farbpigmente auf Basis von mit Eisen(III)oxiden beschichteten Substraten, dadurch gekennzeichnet, daß die Eisenoxidschicht mit Zirkoniumdioxid und Siliziumdioxid dotiert ist.

2. Farbpigmente nach Anspruch 1, dadurch gekennzeichnet, daß der Anteil an Zirkoniumdioxid und Siliziumdioxid in der Eisenoxidschicht 0,1 bis 10 Gew.% beträgt.

3. Farbpigmente nach Anspruch 1 oder 2, dadurch gekennzeichnet, daß das Verhältnis ZrO₂ zu SiO₂ 10:1 bis 0,1:1 beträgt.

4. Farbpigmente nach einem der Ansprüche 1 bis 3, dadurch gekennzeichnet, daß das zu beschichtende Substrat plättchenförmig ist.

5. Farbpigmente nach Anspruch 4, dadurch gekennzeichnet, daß die Substrate Glimmerplättchen, SiO₂-, Glas- oder Keramikflakes sind.

6. Verfahren zur Herstellung von Farbpigmenten nach Anspruch 1, dadurch gekennzeichnet, daß man zu der wäßrigen Substratsuspension nacheinander oder gleichzeitig eine wäßrige Eisen(III)salzlösung, Zirkoniumsalzlösung und eine wasserlösliche Siliziumverbindung unter Bedingungen zugibt, die zur Abscheidung der entsprechenden Oxide bzw. Hydroxide führen, und anschließend das Pigment abtrennt, wäscht, trocknet und bei Temperaturen > 500 °C glüht.

7. Verwendung der Farbpigmente nach Anspruch 1 in Formulierungen wie Lacken, Farbstoffen, Kunststoffen und in kosmetischen Zubereitungen.

8. Verwendung der Farbpigmente nach Anspruch 1 zur Pigmentierung von Wasserlacksystemen.

9. Formulierungen enthaltend Farbpigmente nach Anspruch 1.
